# EUROPEAN PATENT SPECIFICATION

(11) **EP 3 165 248 B1**
(45) Date of publication and mention of the grant of the patent: **31.07.2024**
(21) Application number: 16196661.9
(22) Date of filing: 31.10.2016
(51) Int. Cl.: A61M 5/315, G16H 40/63, G16H 20/17, G16H 20/10, G16H 40/60, A61M 5/31, A61M 5/168

(54) **MEDICAL DEVICE COMPARING ACTUAL AND INTENDED DOSAGE VOLUME**
MEDIZINISCHE VORRICHTUNG ZUM VERGLEICH ZWISCHEN VORGESEHENEM UND EFFEKTIVEM DOSIERUNGSVOLUMEN
DISPOSITIF MÉDICAL POUR COMPARER LE VOLUME DU DOSAGE ACTUEL ET PRÉVU

(30) Priority: 09.11.2015 US 201514936170
(43) Date of publication of application: 10.05.2017
(73) Proprietor: NXP B.V., 5656 AG Eindhoven (NL)
(72) Inventor: DE LOORE, Bart, 5656 AG Eindhoven (NL); FREDERIX, Filip, 5656 AG Eindhoven (NL)
(74) Representative: Miles, John Richard

(56) References cited:
- WO-A2-2011/097487
- US-A1- 2014 094 770

## Description

The present specification relates to systems, methods, apparatuses, devices, articles of manufacture and instructions for patient care.

Medical patients undergo a variety of care. Such care often includes monitoring a patient's current medical condition and administering remedies. For example, diabetic patients require their glucose to be monitored and insulin to be injected as necessary to maintain a target blood glucose level range.

In one example, a diabetic patient first checks their blood glucose level using a glucose meter. Then the patient consults a table to determine how much insulin to inject. Next the patient injects the insulin, perhaps with an insulin pen.

Depending upon the needs of a particular patient, the glucose meter, the table and the insulin pen are on hand to perform this procedure. Depending upon the patient's activity level and food intake, this procedure may need to be frequently performed.

US 2014/094770 A1 discloses a closed-loop feedback control system to ensure accurate drug delivery. WO 2011/097487 A2 discloses a fill adapter device including a heat exchanger which includes a heating element and a fluid pathway, the fluid pathway fluidly connected to a filling needle input, whereby fluid enters the heat exchanger through the filling needle input and flows through the fluid pathway and whereby the fluid is heated by the heating element.

### SUMMARY

According to the invention, a handheld medical device, comprising: a patient attribute sensor input configured to receive a patient attribute input signal; a dosage calculator configured to output a prescribed dosage volume output signal in response to the patient attribute input signal; a dosage volume sensor input configured to receive an actual dosage volume input signal; and a dosage comparator configured to generate a comparator output signal based on a comparison between the prescribed dosage volume output signal and the actual dosage volume input signal.

In another example embodiment, the handheld medical device includes both a patient attribute sensor and a dosage injector.

In another example embodiment, the patient attribute input signal is a patient glucose level input signal; and the dosage injector is an insulin injector.

In another example embodiment, including a patient attribute sensor configured to directly sense at least one of: glucose; a biomarker; or a molecule.

According to the invention the handheld medical device further comprises a dose reservoir, for long term storage of a set of doses; a dose chamber, coupled to the dose reservoir, for short term storage of one of the doses; a dosage volume sensor, coupled to the dose chamber and configured to output the actual dosage volume input signal based on the one of the doses; and an injector configured to administer the one of the doses.

Furthermore, the dosage volume sensor includes a set of capacitive electrodes configured to generate the actual dosage volume input signal.

According to the invention the dose chamber is a syringe; and the injector is a needle.

In another example embodiment, the medical device is a single-use disposable device.

According to the invention the comparator output signal is at least one of: an alarm signal or a display signal.

According to the invention the dosage comparator is configured to generate the comparator output signal if at least one of: the actual dosage volume input signal is below the prescribed dosage volume output signal; or the actual dosage volume input signal is above the prescribed dosage volume output signal.

In another example embodiment, further comprising an output display coupled to receive the comparator output signal.

In another example embodiment, the medical device is remotely powered by at least one of: a wireless signal or an NFC signal.

In another example embodiment, the medical device is paired over a communications link with at least one of: a smartphone; a wearable device; or cloud service.

According to another example embodiment, a medical service for configuring a handheld medical device: wherein the medical device includes: a patient attribute sensor; and a dosage volume sensor, coupled to a dosage chamber; wherein the service includes: receiving a patient attribute input signal from the patient attribute sensor; outputting a prescribed dosage volume output signal in response to the patient attribute input signal; receiving an actual dosage volume input signal from the dosage volume sensor; and generating a comparator output signal based on a comparison between the prescribed dosage volume output signal and the actual dosage volume input signal.

In another example embodiment, the medical service is provided over at least one of: a wireless link; a cloud service; or a smartphone app.

According to a third example embodiment, an article of manufacture including at least one non-transitory, tangible machine readable storage medium containing executable machine instructions for dosage management, comprising: wherein the article includes, a handheld medical device having a patient attribute sensor; and a dosage volume sensor, coupled to a dosage chamber; wherein the instructions include: receiving a patient attribute input signal from the patient attribute sensor; outputting a prescribed dosage volume output signal in response to the patient attribute input signal; receiving an actual dosage volume input signal from the dosage volume sensor; and generating a comparator output signal based on a comparison between the prescribed dosage volume output signal and the actual dosage volume input signal.

The above discussion is not intended to represent every example embodiment or every implementation within the scope of the current or future Claim sets. The Figures and Detailed Description that follow also exemplify various example embodiments.

Various example embodiments may be more completely understood in consideration of the following Detailed Description in connection with the accompanying Drawings, in which:

### BRIEF DESCRIPTION OF THE DRAWINGS

Figure 1 is an example first embodiment of a medical device.
Figure 2 is an example second embodiment of the medical device.
Figure 3 is an example third embodiment of the medical device.
Figure 4 is an example fourth embodiment of the medical device.
Figure 5 is an example list of instructions for enabling the medical device.
Figure 6 is an example system for hosting instructions for enabling the medical device.

While the disclosure is amenable to various modifications and alternative forms, specifics thereof have been shown by way of example in the drawings and will be described in detail. It should be understood, however, that other embodiments, beyond the particular embodiments described, are possible as well. All modifications, equivalents, and alternative embodiments falling within the scope of the appended claims are covered as well.

### DETAILED DESCRIPTION

In one example discussed herein, a handheld medical device for measuring a patient attribute (e.g. glucose level), prescribing a patient dosage amount (e.g. an amount of insulin to be injected, and verifying a dosage amount actually to be administered is discussed.

In one example, the handheld medical device first measures a patient's glucose level in their blood, urine, tears, or any other body fluid. Second, the medical device calculates an amount of insulin to inject, perhaps using a digitally stored look-up table. Third, the device measures an amount of insulin level in a syringe that the patient will actually use to inject the insulin.

Such a device can either be reusable or one-time use. Advantages of such a medical device include: only one device is required for glucose measurement (e.g. meter/reader) and dosage (e.g. injection volume) measurement; handheld device has a smaller form-factor; the bill of materials is reduced; and algorithms in the device can tell a user/patient immediately how much s/he should inject without requiring the user to consult a dosage table, thereby reducing a human error component.

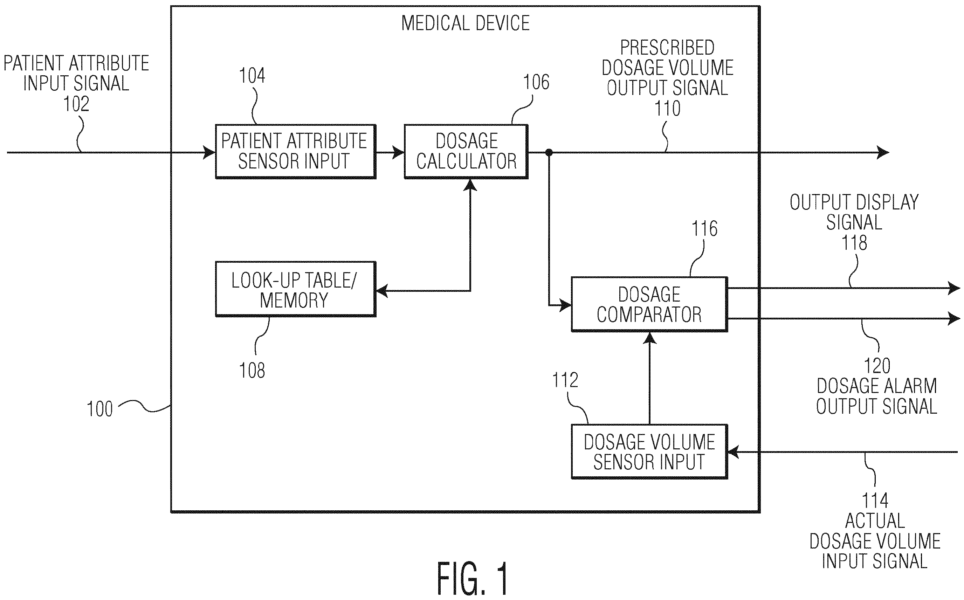

**Figure 1** is an example first embodiment of a medical device 100. The medical device 100, perhaps embodied on a single chip, receives a patient attribute input signal 102 representative of a patient attribute.

The patient attribute input signal 102 in various examples is representative of: a glucose level; a biomarker level; or a molecule level. The patient attribute input signal 102 in some example embodiments is not a user keypad or user input device which manually receives data from a patient or other user.

The patient attribute input signal 102 is received by a patient attribute sensor input 104. A dosage calculator 106 is configured to output a ***prescribed*** dosage volume output signal 110 in response to the patient attribute input signal 102. The dosage calculator 106 may generate the prescribed dosage volume output signal 110 using a look-up table/memory 108.

The prescribed dosage volume output signal 110 corresponds to an amount or volume of a dose to be administered to a patient. Such dose in one example is an amount of a fluid, such as insulin, to be injected in response to a patient's blood glucose level.

A dosage volume sensor input 112 is configured to receive an ***actual*** dosage volume input signal 114 (e.g. perhaps corresponding to an injection volume).

A dosage comparator 116 is configured to generate one or more comparator output signals 118, 120 based on a comparison between the ***prescribed*** dosage volume output signal and the ***actual*** dosage volume input signal. The comparator output signal in various examples can be an output display signal 118, a dosage alarm output signal 120, or both.

If the comparator output signal is an output display signal 118, an output display (not shown) can receive the signal 118 and display to a patient both a prescribed dosage volume and an actual dosage volume, so that the patient can see that the two volumes are approximately equal.

In other examples, when the comparator output signal is a dosage alarm output signal 120, the dosage alarm output signal 120 is generated if at least one of: the actual dosage volume input signal 114 is ***below*** the prescribed dosage volume output signal 110; or the actual dosage volume input signal 114 is ***above*** the prescribed dosage volume output signal 110.

The medical device 100 can in various example embodiments any combination of: a standalone device having a patient attribute reader, a dosage device, an LCD display and a battery; either a single-use-disposable device or a re-useable device; paired with a smartphone or cloud service over a communications link where the medical device 100 does not need to have an output display or perhaps even a battery; remotely powered by either a wireless signal or an NFC signal; include flexible components enabling a user to wear the device, such as during exercise.

In a paired configuration, the medical device 100 can be combined with a medical *service* provider that exchanges data with the medical device 100. The medical service provider in one example: receives the patient attribute input signal 102 from a patient attribute sensor; outputs the ***prescribed*** dosage volume output signal 110 in response to the patient attribute input signal 102; receives the ***actual*** dosage volume input signal 114 from a dosage volume sensor; and generates the comparator output signal 118, 120 based on a comparison between the ***prescribed*** dosage volume output signal 110 and the ***actual*** dosage volume input signal 114. The medical service can in various examples be provided over a wireless link, a cloud service, or a smartphone app.

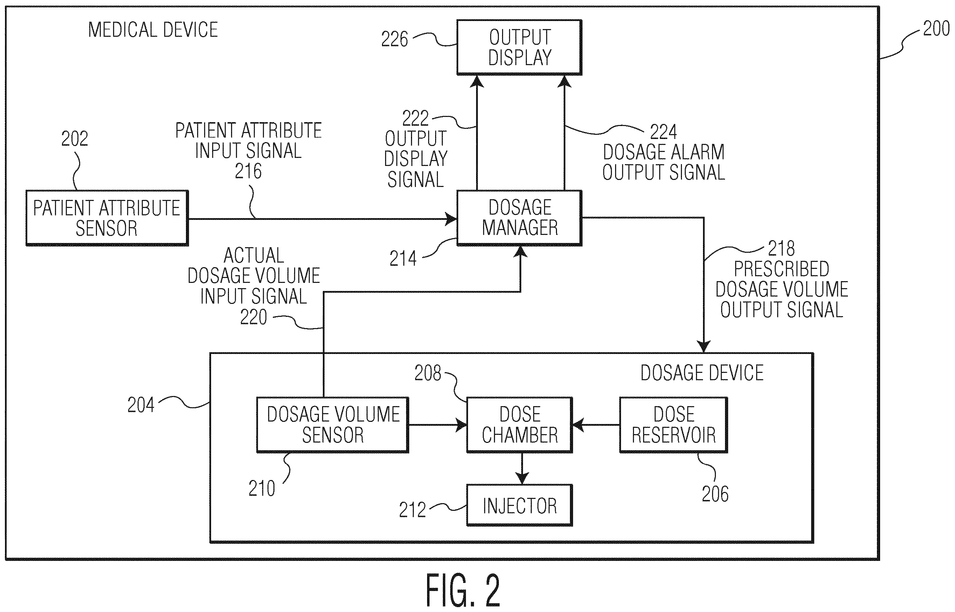

**Figure 2** is an example second embodiment of the medical device 200. The device 200 includes a patient attribute sensor 202. The patient attribute sensor 202 is configured to ***directly sense*** at least one of: glucose; a biomarker; or a molecule.

A dosage device 204 includes a dose reservoir 206 (e.g. for long term does storage), a dose chamber 208 (e.g. for short term dose storage just prior to administration/injection), a dosage volume sensor 210, and an injector 212 (i.e. dosage administration device). The dose chamber 208 receives the dose from the dose reservoir 206.

A dosage manager 214 receives a patient attribute input signal 216 from the patient attribute sensor 202 and generates a prescribed dosage volume output signal 218 in a manner similar to that discussed with respect to the dosage calculator 106 in Figure 1.

The dosage device 204 receives the prescribed dosage volume output signal 218 and meters a dose transfer from the dose reservoir 206 to the dose chamber 208. The dosage volume sensor 210 generates an actual dosage volume input signal 220 from a set of capacitive electrodes *(not shown)* positioned around the dose chamber 208. Additional capacitive electrodes can be placed around the dose reservoir 206 indicating to a patient when a new dose reservoir 206 is required.

The dosage manager 214 transmits an output display signal 222 and/or a dosage alarm output signal 224 to an output display 226. Example information in the output display signal 222 and triggers for the dosage alarm output signal 224 have been discussed with respect to Figure 1.

When conditions are right the dosage manager 214 sends a signal to the dosage device 204 to administer the dose in the dose chamber 208 using the injector 212. Such conditions could include a patient's permission signal or automatically when the actual dosage volume input signal 220 is equal to the prescribed dosage volume output signal 218.

In one example, the medical device 200 can be a combination glucose monitor and insulin pen, where the patient attribute input signal 216 is a patient glucose level input signal, and the dosage device 204 is the insulin pen. The dosage device 204 in an example is a syringe with a tube, plunger, and injection needle.

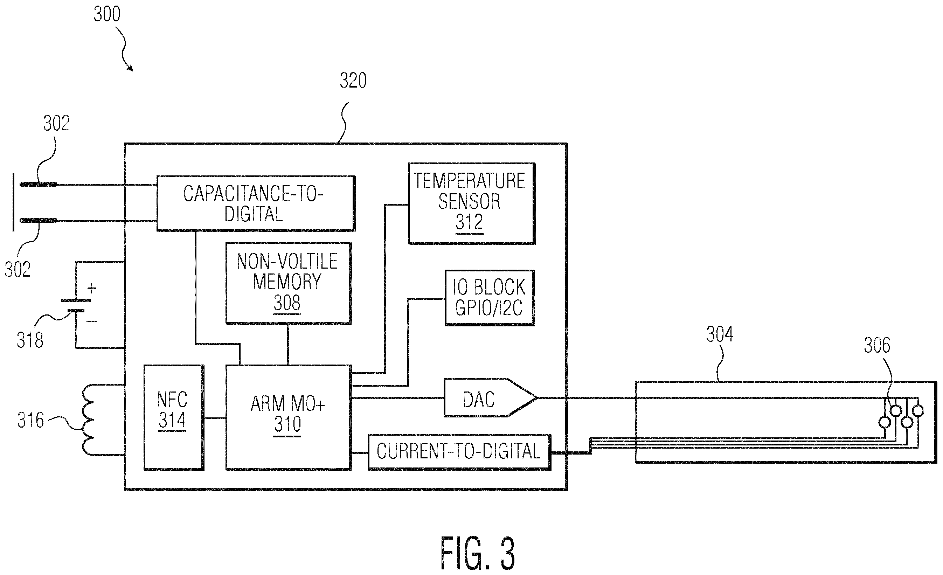

**Figure 3** is an example third embodiment of the medical device 300. This example medical device 300 shows a first set of electrodes 306 (e.g. an amperiometric sensor) on a disposable strip 304 for generating a patient attribute input signal (e.g. 102, 216), such as glucose level.

A second set of capacitive electrodes 302, in a dose chamber *(not shown),* for generating an actual dosage volume input signal (e.g. 114, 220). The device 300 includes a non-volatile memory 308 which can be used to store a look-up table for matching the patient attribute input signal with a prescribed dosage volume output signal.

The device 300 includes an ARM microcontroller 310, temperature sensor 312, a wireless interface 314 (e.g. NFC), an antenna 316, and a battery 318. In this example, all device 300 components except the first electrodes 306, disposable strip 304, second electrodes 302, dose chamber, antenna 316, and battery 318 are mounted within a single chip processor 320.

The single chip processor 320 can also include a real-time clock and a capacitance-to-digital circuit connected to the second set of capacitive electrodes 302, which as previously mentioned reads the amount of insulin dose in the dose chamber.

The wireless interface 314 in one example communicates with either a cloud service or database containing patient history information and routines for calculating a dosage.

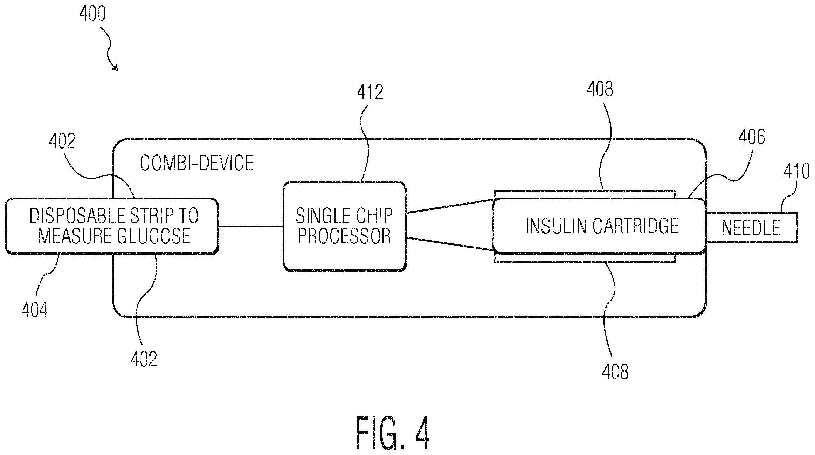

**Figure 4** is an example fourth embodiment of the medical device 400. The fourth example medical device 400 includes an input interface 402 for receiving a disposable strip 404 (e.g. blood glucose strip). The medical device 400 also includes an insulin cartridge 406 having capacitive electrodes 408 for volume measurement. The insulin cartridge 406 is connected to a needle 410 for injecting a dose, such as insulin.

A single chip processor 412 processes signals from the input interface 402 and the capacitive electrodes 408 and generates a prescribed dosage volume output signal for display *(not shown).*

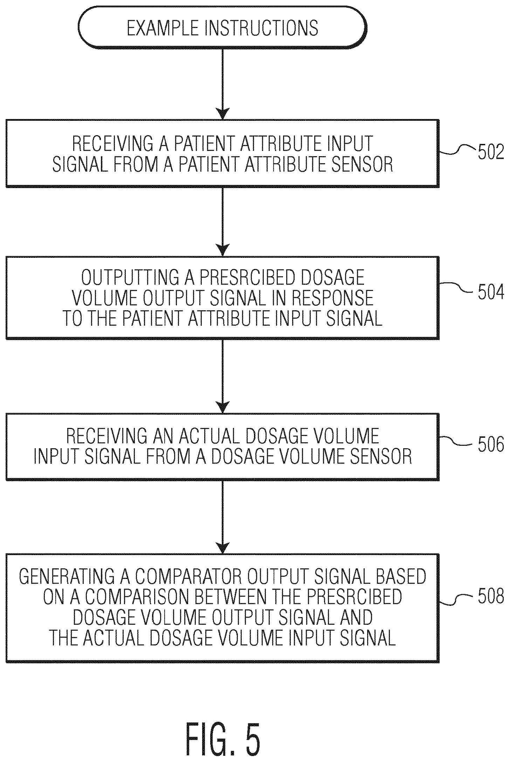

**Figure 5** is an example list of instructions for enabling the medical device. The order in which the instructions are discussed does not limit the order in which other example embodiments implement the instructions unless otherwise specifically stated. Additionally, in some embodiments the instructions are implemented concurrently.

A first example instruction set begins in 502, by receiving a patient attribute input signal from a patient attribute sensor. Next, in 504, outputting a prescribed dosage volume output signal in response to the patient attribute input signal. Then in 506, receiving an actual dosage volume input signal from a dosage volume sensor. In 508, generating a comparator output signal based on a comparison between the prescribed dosage volume output signal and the actual dosage volume input signal.

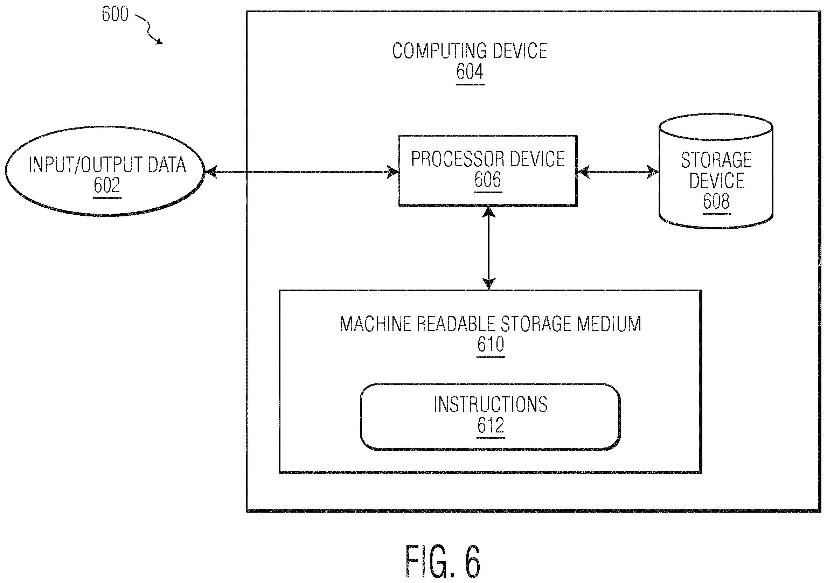

**Figure 6** is an example system 600 for hosting instructions for enabling the medical device. The system 600 shows an input/output data 602 interface with an electronic apparatus 604. The electronic apparatus 604 includes a processor 606, a storage device 608, and a non-transient machine-readable storage medium 610. The machine-readable storage medium 610 includes instructions 612 which control how the processor 606 receives input data 602 and transforms the input data into output data 602, using data within the storage device 608. Example instructions 612 stored in the machine-readable storage medium 610 are discussed elsewhere in this specification. The machine-readable storage medium in an alternate example embodiment is a non-transient computer-readable storage medium.

The processor (such as a central processing unit, CPU, microprocessor, application-specific integrated circuit (ASIC), etc.) controls the overall operation of the storage device (such as random access memory (RAM) for temporary data storage, read only memory (ROM) for permanent data storage, firmware, flash memory, external and internal hard-disk drives, and the like). The processor device communicates with the storage device and non-transient machine-readable storage medium using a bus and performs operations and tasks that implement one or more instructions stored in the machine-readable storage medium. The machine-readable storage medium in an alternate example embodiment is a computer-readable storage medium.

The instructions and/or flowchart steps in the above Figures can be executed in any order, unless a specific order is explicitly stated. Also, those skilled in the art will recognize that while one example set of instructions/method has been discussed, the material in this specification can be combined in a variety of ways to yield other examples as well, and are to be understood within a context provided by this detailed description.

In some example embodiments the set of instructions described above are implemented as functional and software instructions embodied as a set of executable instructions in a non-transient computer-readable or computer-usable media which are effected on a computer or machine programmed with and controlled by said executable instructions. Said instructions are loaded for execution on a processor (such as one or more CPUs). Said processor includes microprocessors, microcontrollers, processor modules or subsystems (including one or more microprocessors or microcontrollers), or other control or computing devices. A processor can refer to a single component or to plural components. Said computer-readable or computer-usable storage medium or media is (are) considered to be part of an article (or article of manufacture). An article or article of manufacture can refer to any manufactured single component or multiple components. The non-transient machine or computer-usable media or mediums as defined herein excludes signals, but such media or mediums may be capable of receiving and processing information from signals and/or other transient mediums.

Example embodiments of the material discussed in this specification can be implemented in whole or in part through network, computer, or data based devices and/or services. These may include cloud, internet, intranet, mobile, desktop, processor, look-up table, microcontroller, consumer equipment, infrastructure, or other enabling devices and services. As may be used herein and in the claims, the following non-exclusive definitions are provided.

In this specification, example embodiments have been presented in terms of a selected set of details. However, a person of ordinary skill in the art would understand that many other example embodiments may be practiced which include a different selected set of these details. It is intended that the following claims cover all possible example embodiments.

## Claims

1. A handheld medical device (100), comprising:
a patient attribute sensor input (104) configured to receive a patient attribute input signal (102);
a dosage calculator (106) configured to output a prescribed dosage volume output signal (110) in response to the patient attribute input signal;
a dosage volume sensor input (112) configured to receive an actual dosage volume input signal (114, 220); and
a dose reservoir (206), for long term storage of a set of doses;
a dose chamber (208), coupled to the dose reservoir, for short term storage of one of the doses, wherein the dose chamber is a syringe;
a dosage volume sensor (210), coupled to the dose chamber and configured to output the actual dosage volume input signal (114, 220) based on the one of the doses, wherein the dosage volume sensor (210) includes a set of capacitive electrodes configured to generate the actual dosage volume input signal (114, 220); and
an injector (212) configured to administer the one of the doses stored in the dose chamber, wherein the injector is a needle;
a dosage comparator (116) configured to generate a comparator output signal (118, 120) based on a comparison between the prescribed dosage volume output signal and the actual dosage volume input signal;
wherein the comparator output signal is at least one of: an alarm signal (120) or a display signal (118); and
wherein the dosage comparator is configured to generate the comparator output signal if at least one of: the actual dosage volume input signal is below the prescribed dosage volume output signal; or the actual dosage volume input signal is above the prescribed dosage volume output signal.

2. The device (200) of claim 1:
wherein the handheld medical device includes a patient attribute sensor (202).

3. The device (200) of claim 2:
wherein the patient attribute input signal (216) is a patient glucose level input signal; and
wherein the dosage injector (212) is an insulin injector.

4. The device (200) of any preceding claim, wherein:
the patient attribute sensor (202) is configured to directly sense at least one of: glucose; a biomarker; or a molecule.

5. The device (200) of any preceding claim:
wherein the medical device is a single-use disposable device.

6. The device (200) of any preceding claim:
further comprising an output display (226) coupled to receive the comparator output signal (222).

7. The device (200) of any preceding claim:
wherein the medical device is remotely powered by at least one of: a wireless signal or an NFC signal.

8. The device (200) of any preceding claim:
wherein the medical device is paired over a communications link with at least one of: a smartphone; a wearable device; or cloud service.

9. The device (200) of any preceding claim, wherein:
the patient attribute sensor input (104) is configured to be coupled to a first set of electrodes on a disposable strip for generating a patient attribute input signal; and
the dosage volume sensor input (112) comprises a capacitance-to-digital circuit configured to be connected to the set of capacitive electrodes; and
the patient attribute sensor input (104), the dosage calculator (106), the dosage volume sensor input (112) and the dosage comparator (116) are within a single chip processor.

10. A computer implemented method for configuring a handheld medical device:
wherein the medical device includes:
a patient attribute sensor; and
a dosage volume sensor, coupled to a dosage chamber and configured to output the actual dosage volume input signal (220) based on the one of the doses, wherein the dose chamber is a syringe configured to provide short term storage of one of the doses and wherein the dosage volume sensor (210) includes a set of capacitive electrodes configured to generate the actual dosage volume input signal (220); and
an injector (212) configured to administer the one of the doses stored in the dose chamber, wherein the injector is a needle;
wherein the computer implemented method includes:
receiving (502) a patient attribute input signal from the patient attribute sensor;
outputting (504) a prescribed dosage volume output signal in response to the patient attribute input signal;
receiving (506) an actual dosage volume input signal from the dosage volume sensor; and
generating (508) a comparator output signal based on a comparison between the prescribed dosage volume output signal and the actual dosage volume input signal; and
wherein the comparator output signal is at least one of: an alarm signal or a display signal; and
wherein the dosage comparator is configured to generate the comparator output signal if at least one of: the actual dosage volume input signal is below the prescribed dosage volume output signal; or the actual dosage volume input signal is above the prescribed dosage volume output signal.

11. The computer implemented method of claim 10:
wherein the computer implemented method is provided over at least one of: a wireless link; a cloud service; or a smartphone app.

## Patentansprüche

1. Handgeführte medizinische Vorrichtung (100), umfassend:
einen Patientenmerkmalsensoreingang (104), der konfiguriert ist, um ein Patientenmerkmaleingangssignal (102) zu empfangen;
einen Dosierungsrechner (106), der konfiguriert ist, um in Reaktion auf das Patientenmerkmaleingangssignal ein Ausgangssignal (110) eines verordneten Dosierungsvolumens auszugeben
einen Dosierungsvolumensensoreingang (112), der konfiguriert ist, um ein Eingangssignal (114, 220) eines tatsächlichen Dosierungsvolumens zu empfangen; und
ein Dosisreservoir (206) zur langfristigen Lagerung eines Satzes von Dosen;
eine Dosiskammer (208), die an das Dosisreservoir gekoppelt ist, zur kurzfristigen Lagerung von einer der Dosen, wobei die Dosiskammer eine Spritze ist;
einen Dosierungsvolumensensor (210), der an die Dosiskammer gekoppelt ist und konfiguriert ist, um das Eingangssignal (114, 220) des tatsächlichen Dosierungsvolumens basierend auf der einen der Dosen auszugeben, wobei der Dosierungsvolumensensor (210) einen Satz von kapazitiven Elektroden einschließt, der konfiguriert ist, um das Eingangssignal (114, 220) des tatsächlichen Dosierungsvolumens zu generieren; und
einen Injektor (212), der konfiguriert ist, um die eine der in der Dosiskammer gelagerten Dosen zu verabreichen, wobei der Injektor eine Nadel ist;
einen Dosierungskomparator (116), der konfiguriert ist, um ein Komparatorausgangssignal (118, 120) basierend auf einem Vergleich zwischen dem Ausgangssignal des verordneten Dosierungsvolumens und dem Eingangssignal des tatsächlichen Dosierungsvolumens zu generieren;
wobei das Komparatorausgangssignal mindestens eines von einem Alarmsignal (120) oder einem Anzeigesignal (118) ist; und
wobei der Dosierungskomparator konfiguriert ist, um das Komparatorausgangssignal zu generieren, falls mindestens eines der folgenden zutrifft: das Eingangssignal des tatsächlichen Dosierungsvolumens ist unter dem Ausgangssignal des verordneten Dosierungsvolumens; oder
das Eingangssignal des tatsächlichen Dosierungsvolumens ist oberhalb des Ausgangssignals des verordneten Dosierungsvolumens.

2. Vorrichtung (200) nach Anspruch 1:
wobei die handgeführte medizinische Vorrichtung einen Patientenmerkmalsensor (202) einschließt.

3. Vorrichtung (200) nach Anspruch 2:
wobei das Patientenmerkmaleingangssignal (216) ein Eingangssignal des Patientenglucosespiegels ist; und
wobei der Dosierungsinjektor (212) ein Insulininjektor ist.

4. Vorrichtung (200) nach einem vorhergehenden Anspruch, wobei:
der Patientenmerkmalsensor (202) konfiguriert ist, um mindestens eines von Glucose; einem Biomarker oder einem Molekül direkt abzufühlen.

5. Vorrichtung (200) nach einem vorhergehenden Anspruch:
wobei die medizinische Vorrichtung eine Wegwerfvorrichtung zum einmaligen Gebrauch ist.

6. Vorrichtung (200) nach einem vorhergehenden Anspruch:
ferner umfassend eine Ausgabeanzeige (226), die gekoppelt ist, um das Komparatorausgangssignal (222) zu empfangen.

7. Vorrichtung (200) nach einem vorhergehenden Anspruch:
wobei die medizinische Vorrichtung aus der Ferne angetrieben wird durch mindestens eines von einem drahtlosen Signal oder einem NFC-Signal.

8. Vorrichtung (200) nach einem vorhergehenden Anspruch:
wobei die medizinische Vorrichtung über eine Kommunikationsverbindung mit mindestens einem von einem Smartphone; einer Wearable-Vorrichtung oder einem Cloud-Dienst gepaart ist.

9. Vorrichtung (200) nach einem vorhergehenden Anspruch, wobei:
der Patientenmerkmalsensoreingang (104) konfiguriert ist, um an einen ersten Satz von Elektroden auf einem Wegwerfstreifen gekoppelt zu werden, um ein Patientenmerkmaleingangssignal zu generieren; und
der Dosierungsvolumensensoreingang (112) eine Kapazitätzu-Digital-Schaltung umfasst, die konfiguriert ist, um mit dem Satz der kapazitiven Elektroden verbunden zu werden; und
der Patientenmerkmalsensoreingang (104), der Dosierungsrechner (106), der Dosierungsvolumensensoreingang (112) und der Dosierungskomparator (116) sich innerhalb eines Einchipprozessors befinden.

10. Computerimplementiertes Verfahren zum Konfigurieren einer handgeführten medizinischen Vorrichtung, wobei die medizinische Vorrichtung einschließt:
einen Patientenmerkmalsensor; und
einen Dosierungsvolumensensor, der an eine Dosierungskammer gekoppelt ist und konfiguriert ist, um das Eingangssignal (220) des tatsächlichen Dosierungsvolumens basierend auf der einen der Dosen auszugeben, wobei die Dosiskammer eine Spritze ist, die konfiguriert ist, um kurzfristige Lagerung von einer der Dosen bereitzustellen, und wobei der Dosierungsvolumensensor (210) einen Satz von kapazitiven Elektroden einschließt, der konfiguriert ist, um das Eingangssignal (220) des tatsächlichen Dosierungsvolumens zu generieren; und
einen Injektor (212), der konfiguriert ist, um die eine der in der Dosiskammer gelagerten Dosen zu verabreichen, wobei der Injektor eine Nadel ist;
wobei das computerimplementierte Verfahren einschließt:
Empfangen (502) eines Patientenmerkmaleingangssignals von dem Patientenmerkmalsensor;
Ausgeben (504) eines Ausgangssignals eines verordneten Dosierungsvolumens in Reaktion auf das Patientenmerkmaleingangssignal;
Empfangen (506) eines Eingangssignals des tatsächlichen Dosierungsvolumens von dem Dosierungsvolumensensor; und
Generieren (508) eines Komparatorausgangssignals basierend auf einem Vergleich zwischen dem Ausgangssignal des verordneten Dosierungsvolumens und dem Eingangssignal des tatsächlichen Dosierungsvolumens; und
wobei das Komparatorausgangssignal mindestens eines von einem Alarmsignal oder einem Anzeigesignal ist; und
wobei der Dosierungskomparator konfiguriert ist, um das Komparatorausgangssignal zu generieren, falls mindestens eines der folgenden zutrifft: das Eingangssignal des tatsächlichen Dosierungsvolumens ist unter dem Ausgangssignal des verordneten Dosierungsvolumens; oder das Eingangssignal des tatsächlichen Dosierungsvolumens ist oberhalb des Ausgangssignals des verordneten Dosierungsvolumens.

11. Computerimplementiertes Verfahren nach Anspruch 10: wobei das computerimplementierte Verfahren über mindestens eines von einer drahtlosen Verbindung; einem Cloud-Dienst oder einer Smartphone-App bereitgestellt wird.

## Revendications

1. Dispositif médical portable (100), comprenant :
une entrée de capteur d'attributs de patient (104) configurée pour recevoir un signal d'entrée d'attributs de patient (102) ;
un calculateur de dosage (106) configuré pour émettre un signal de sortie de volume de dosage prescrit (110) en réponse au signal d'entrée d'attribut de patient ;
une entrée de capteur de volume de dosage (112) configurée pour recevoir un signal d'entrée de volume de dosage actuel (114, 220) ; et
un réservoir de doses (206), pour le stockage à long terme d'un ensemble de doses ;
une chambre de dose (208), couplée au réservoir de dose, pour le stockage à court terme de l'une des doses, la chambre de dose étant une seringue ;
un capteur de volume de dosage (210), couplé à la chambre de dose et configuré pour émettre le signal d'entrée de volume de dosage actuel (114, 220) sur la base de l'une des doses, le capteur de volume de dosage (210) comprenant un ensemble d'électrodes capacitives configurées pour générer le signal d'entrée de volume de dosage actuel (114, 220) ; et
un injecteur (212) configuré pour administrer l'une des doses stockées dans la chambre de dose, l'injecteur étant une aiguille ;
un comparateur de dosage (116) configuré pour générer un signal de sortie de comparateur (118, 120) basé sur une comparaison entre le signal de sortie de volume de dosage prescrit et le signal d'entrée de volume de dosage actuel ;
le signal de sortie de comparateur étant au moins l'un des signaux suivants : un signal d'alarme (120) ou un signal d'affichage (118) ; et
le comparateur de dosage étant configuré pour générer le signal de sortie de comparateur si au moins l'un des éléments suivants : le signal d'entrée de volume de dosage actuel est inférieur au signal de sortie de volume de dosage prescrit ; ou le signal d'entrée de volume de dosage actuel est supérieur au signal de sortie de volume de dosage prescrit.

2. Dispositif (200) selon la revendication 1 :
le dispositif médical portable comprenant un capteur d'attributs de patient (202).

3. Dispositif (200) selon la revendication 2 :
le signal d'entrée d'attribut de patient (216) étant un signal d'entrée de niveau de glucose de patient ; et
l'injecteur de dose (212) étant un injecteur d'insuline.

4. Dispositif (200) selon n'importe quelle revendication précédente,
le capteur d'attributs de patient (202) étant configuré pour détecter directement au moins l'un des éléments suivants : du glucose, un biomarqueur ou une molécule.

5. Dispositif (200) selon n'importe quelle revendication précédente :
le dispositif médical étant un dispositif jetable à usage unique.

6. Dispositif (200) selon n'importe quelle revendication précédente :
comprenant en outre un affichage de sortie (226) couplé pour recevoir le signal de sortie de comparateur (222).

7. Dispositif (200) selon n'importe quelle revendication précédente :
le dispositif médical étant alimenté à distance par au moins l'un des éléments suivants : un signal sans fil ou un signal NFC.

8. Dispositif (200) selon n'importe quelle revendication précédente :
le dispositif médical étant apparié par un lien de communication avec au moins l'un parmi : un smartphone ; un dispositif portable ; ou un service en nuage.

9. Dispositif (200) selon n'importe quelle revendication précédente,
l'entrée de capteur d'attributs de patient (104) étant configurée pour être couplée à un premier ensemble d'électrodes sur une bande jetable afin de générer un signal d'entrée d'attributs de patient ; et
l'entrée de capteur de volume de dosage (112) comprenant un circuit capacitif-numérique configuré pour être connecté à l'ensemble d'électrodes capacitives ; et
l'entrée de capteur d'attributs de patient (104), le calculateur de dosage (106), l'entrée de capteur de volume de dosage (112) et le comparateur de dosage (116) étant à l'intérieur d'un processeur à puce unique.

10. Procédé mis en œuvre par ordinateur pour configurer un dispositif médical portable, le dispositif médical comprenant :
un capteur d'attributs de patient ; et
un capteur de volume de dosage, couplé à une chambre de dosage et configuré pour émettre le signal d'entrée de volume de dosage actuel (220) en fonction de l'une des doses, la chambre de dose étant une seringue configurée pour fournir un stockage à court terme de l'une des doses et le capteur de volume de dosage (210) comprenant un ensemble d'électrodes capacitives configurées pour générer le signal d'entrée de volume de dosage actuel (220) ; et
un injecteur (212) configuré pour administrer l'une des doses stockées dans la chambre de dose, l'injecteur étant une aiguille ;
le procédé mis en œuvre par ordinateur comprenant :
la réception (502) d'un signal d'entrée d'attributs de patient provenant du capteur d'attributs de patient ;
l'émission (504) d'un signal de sortie de volume de dosage prescrit en réponse au signal d'entrée d'attribut de patient ;
la réception (506) d'un signal d'entrée de volume de dosage actuel provenant du capteur de volume de dosage ; et
la génération (508) d'un signal de sortie de comparateur basé sur une comparaison entre le signal de sortie de volume de dosage prescrit et le signal d'entrée de volume de dosage actuel ; et
le signal de sortie de comparateur étant au moins l'un des signaux suivants : un signal d'alarme ou un signal d'affichage ; et
le comparateur de dosage étant configuré pour générer le signal de sortie de comparateur si au moins l'un des éléments suivants : le signal d'entrée de volume de dosage actuel est inférieur au signal de sortie de volume de dosage prescrit ; ou le signal d'entrée de volume de dosage actuel est supérieur au signal de sortie de volume de dosage prescrit.

11. Procédé mis en œuvre par ordinateur selon la revendication 10 :
le procédé mis en œuvre par ordinateur étant fourni sur au moins l'un des éléments suivants : une liaison sans fil ; un service en nuage ; ou une application pour smartphone.
